(19) Europäisches Patentamt
European Patent Office
Office européen des brevets

(11) Veröffentlichungsnummer : **0 513 134 B1**

# (12) EUROPÄISCHE PATENTSCHRIFT

(45) Veröffentlichungstag der Patentschrift :
**17.08.94 Patentblatt 94/33**

(51) Int. Cl.$^5$ : **C11D 1/14,** C11D 1/16, C07C 305/10

(21) Anmeldenummer : **91903644.2**

(22) Anmeldetag : **24.01.91**

(86) Internationale Anmeldenummer :
**PCT/EP91/00135**

(87) Internationale Veröffentlichungsnummer :
**WO 91/11504 08.08.91 Gazette 91/18**

## (54) SULFIERTE HYDROXYCARBONSÄUREESTER.

(30) Priorität : **02.02.90 DE 4003096**

(43) Veröffentlichungstag der Anmeldung :
**19.11.92 Patentblatt 92/47**

(45) Bekanntmachung des Hinweises auf die Patenterteilung :
**17.08.94 Patentblatt 94/33**

(84) Benannte Vertragsstaaten :
**DE ES FR IT**

(56) Entgegenhaltungen :
**EP-A- 0 193 386**
**EP-A- 0 267 518**
**EP-A- 0 299 370**
**EP-A- 0 366 015**
**CH-A- 477 415**

(73) Patentinhaber : **Henkel Kommanditgesellschaft auf Aktien**
**D-40191 Düsseldorf (DE)**

(72) Erfinder : **Fabry, Bernd Dr.**
**Danziger Strasse 31,**
**W-4052 Korschenbroich 1 (DE)**

EP 0 513 134 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Gegenstand der Erfindung sind anionische Tenside erhältlich aus Hydroxycarbonsäureestern durch Umsetzung mit Sulfiermitteln und anschließende Neutralisation mit wässrigen Basen, ein Verfahren zu ihrer Herstellung sowie ihre Verwendung als oberflächenaktive Substanzen.

Hydroxycarbonsäuren stellen in der Natur weitverbreitete organische Substanzen dar. So entsteht z. B. bei der Gärung von Milch 2-Hydroxypropionsäure (Milchsäure). 2-Hydroxybernsteinsäure (Äpfelsäure) und 2-Hydroxy-1,2,3-propantricarbonsäure (Citronensäure) werden als wichtige Zwischenprodukte im menschlichen Stoffwechsel gebildet. Dem bei der Weinherstellung anfallenden Kaliumhydrogentartrat ("Weinstein") liegt schließlich die 2,3-Dihydroxy-bernsteinsäure (Weinsäure) zugrunde.

Milchsäure wird großtechnisch z. B. durch Fermentation von Stärke gewonnen, die Herstellung von Äpfel- und Citronensäure erfolgt auf biotechnologischem Wege, während man Weinsäure durch klassischen Aufschluß von Weinstein mit Schwefelsäure erhält [Chem.Tech., **21**, 425 (1973), New.Sci., **67**, 538 (1975)].

Die Ester dieser Säuren haben industrielles Interesse als oberflächenaktive Stoffe, beispielsweise als Emulgatoren, Korrosionsinhibitoren, Builder und Nahrungsmittelzusatzstoffe gefunden. So sind z. B. aus der deutschen Patentanmeldung DE-A-36 05 799 Citronensäureester bekannt, die als Emulgatoren in der Emulsionspolymerisation Verwendung finden.

Die beiden japanischen Anmeldungen JP-A-59/190 907 und JP-A-59/25 640 beschreiben die Verwendung von Milch- und Weinsäureestern langkettiger Alkohole als öllösliche Emulgatoren bei der Herstellung von Kosmetika.

Die Patentanmeldungen EP-A-0 008 195, EP-A-0 199 131, EP-A-0 209 910 und EP-A-0 258 814 betreffen ferner die Verwendung von Citronen- bzw. Weinsäureestern in kosmetischen Präparaten.

Hydroxycarbonsäureester sind biologisch leicht abbaubar, toxikologisch unbedenklich und somit prinzipiell auch für die Herstellung von Wasch- und Reinigungsmitteln interessant. Ihre Verwendung scheitert jedoch daran, daß sie in Wasser unzureichend löslich sind.

Der Erfindung lag die Aufgabe zugrunde, Hydroxycarbonsäureesterderivate zu entwickeln, die eine so hohe Wasserlöslichkeit besitzen, daß sie als oberflächenaktive Substanzen für Wasch- und Reinigungsmittel in Betracht kommen.

Gegenstand der Erfindung sind anionische Tenside erhältlich aus Hydroxycarbonsäureestern der allgemeinen Formel **(I)**,

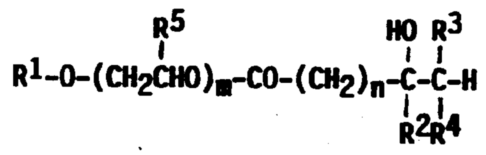

(I)

in der $R^1$ für einen linearen oder verwzweigten aliphatischen Kohlenwasserstoffrest mit 6 bis 22 Kohlenstoffatomen und 0, 1, 2 oder 3 Doppelbindungen, $R^2$ und $R^4$ unabhängig voneinander für Wasserstoff oder eine Gruppe $R^1$-O-($CH_2$-$CHR^5$-O)$_m$-CO-, $R^3$ für Wasserstoff oder eine Hydroxylgruppe, $R^5$ für Wasserstoff oder eine Methylgruppe, n für 0 oder 1 und m für 0 oder eine Zahl von 1 bis 20 stehen, durch Umsetzung mit einem Sulfiermittel und anschließende Neutralisation mit einer wässrigen Base.

Gegenstand der Erfindung ist ferner ein Verfahren zur Herstellung von anionischen Tensiden erhältlich aus Hydroxycarbonsäureestern der allgemeinen Formel **(I)**,

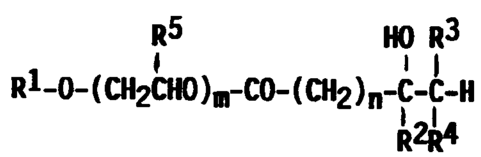

(I)

in der $R^1$ für einen linearen oder verzweigten aliphatischen Kohlenwasserstoffrest mit 6 bis 22 Kohlenstoffatomen und 0, 1, 2 oder 3 Doppelbindungen, $R^2$ und $R^4$ unabhängig voneinander für Wasserstoff oder eine Gruppe $R^1$-O-($CH_2$-$CHR^5$-O)$_m$CO-, $R^3$ für Wasserstoff oder eine Hydroxylgruppe, $R^5$ für Wasserstoff oder eine Methylgruppe, n für 0 oder 1 und m für 0 oder eine Zahl von 1 bis 20 stehen, durch Umsetzung mit einem Sulfiermittel und anschließende Neutralisation mit einer wässrigen Base.

Die Erfindung beruht auf der Erkenntnis, daß Hydroxycarbonsäureester gesättigter oder ungesättigter aliphatischer Alkohole durch Umsetzung mit einem Sulfiermittel und anschließende Neutralisation mit einer

wässrigen Base in anionische Tenside mit hinreichender Wasserlöslichkeit und überraschend hoher dermatologischer Verträglichkeit überführt werden können.

Bei den Ausgangsmaterialien für die Herstellung der erfindungsgemäßen anionischen Tenside handelt es sich um bekannte Verbindungen, die nach gängigen Methoden der organischen Synthese, beispielsweise durch Veresterung von Hydroxycarbonsäuren mit aliphatischen Alkoholen in Gegenwart von üblichen Veresterungskatalysatoren erhalten werden können.

Unter Hydroxycarbonsäureestern sind z. B. Ester der Milchsäure und der Äpfelsäure zu verstehen. Infolge ihrer leichten Verfügbarkeit wird jedoch bevorzugt von Estern der Citronensäure und der Weinsäure ausgegangen.

Der Rest $R^1$ in Formel **(I)** kann sich von linearen oder verzweigten, gesättigten oder ungesättigten Alkoholen mit 6 bis 22 Kohlenstoffatomen und 0, 1, 2 oder 3 Doppelbindungen ableiten. Typische Vertreter sind Capronalkohol, Caprylalkohol, Caprinalkohol, Myristylalkohol, Laurylalkohol, Cetylalkohol, Palmitoleylalkohol, Stearylalkohol, Oleylalkohol, Elaidylalkohol, Petroselylalkohol, Linolylalkohol, Linolenylalkohol, Behenylalkohol oder Erucylalkohol.

Der Rest $R^1$ kann sich auch von Alkoholgemischen ableiten, wie sie etwa bei der Hydrierung technischer Fettsäuremethylesterfraktionen, z. B. auf Basis von Kokosöl, Palmöl, Palmkernöl, Sonnenblumenöl, Rüböl, Korianderöl, Erdnußöl, Leinöl, Lardöl, Rindertalg oder Fischöl anfallen. Gleichfalls geeignet sind Oxoalkoholgemische, wie sie z. B. durch Hydrierung technischer Aldehydschnitte aus der Roelen'schen Oxosynthese zugänglich sind.

Anionische Tenside mit besonders starkem Schaumvermögen sind durch Sulfierung von Hydroxycarbonsäureestern erhältlich, bei denen sich $R^1$ von linearen, gesättigten Alkoholen mit 8 bis 18 Kohlenstoffatomen ableitet.

Anionische Tenside mit besonders hoher Wasserlöslichkeit und guter Hautverträglichkeit sind hingegen durch Sulfierung von Hydroxycarbonsäureestern erhältlich, bei denen sich $R^1$ von ungesättigten Alkoholen mit 16 bis 22 Kohlenstoffatomen und 1, 2 oder 3 Doppelbindungen ableitet.

Als Ausgangsmaterial für die Herstellung der erfindungsgemäßen anionischen Tenside eignen sich ferner Ester von Hydroxycarbonsäuren mit Anlagerungsprodukten von durchschnittlich 1 bis 20 Mol Ethylenoxid, Propylenoxid oder Gemischen beider (in Random- oder Blockverteilung) an die zuvor im Zusammenhang mit $R^1$ genannten Alkohole. Die entsprechenden Alkylenoxidaddukte, die der Formel **(III)** folgen,

$$R^1\text{-}O\text{-}(CH_2\overset{\displaystyle R^5}{\overset{|}{C}}H\text{-}O)_mH \qquad \text{(III)}$$

können nach den an sich bekannten großtechnischen Verfahren, z. B. in Gegenwart von Natriummethylat oder Hydrotalcit als Alkoxylierungskatalysator erhalten werden. Da die Ethoxylierung der Alkohole einem statistischen Verlauf unterliegt, muß der durchschnittliche Ethoxylierungsgrad dabei nicht notwendigerweise eine ganze Zahl darstellen, sondern kann vielmehr auch gebrochene Zahlenwerte - auch solche kleiner 1 - annehmen.

Anionische Tenside mit besonders starkem Schaumvermögen und hoher Wasserlöslichkeit sind ferner durch Sulfierung von Hydroxyfettsäureestern erhältlich, bei denen sich $R^1$ von Anlagerungsprodukten von durchschnittlich 1 bis 20, vorzugsweise 2 bis 5 Mol Ethylenoxid an lineare, gesättigte Alkohole mit 8 bis 18 Kohlenstoffatomen ableitet.

Die Sulfierung der Hydroxycarbonsäureester kann mit üblichen Sulfiermitteln, z. B. Schwefelsäure, Oleum, Chlorsulfonsäure, Amidosulfonsäure, insbesondere aber gasförmigem Schwefeltrioxid im Gemisch mit einem Inertgas erfolgen. Dabei wird das Schwefeltrioxid mit Luft oder Stickstoff verdünnt und vorzugsweise in Form eines Gasgemisches mit ca. 1 bis 8, insbesondere 3 bis 5 Vol.-% Schwefeltrioxid eingesetzt. Die Sulfierung kann ferner kontinuierlich oder diskontinuierlich, insbesondere jedoch in Reaktoren durchgeführt werden, die nach dem Fallfilmprinzip arbeiten.

Bevorzugt wird die Reaktion in Abwesenheit von Lösungsmitteln durchgeführt; es können jedoch auch sämtliche für die Sulfierung von Olefinen, Aromaten, Alkoholen und dergleichen üblichen Lösungsmittel wie z. B. Orthoameisensäureester, Dimethylformamid, 1.2-Dichlorethan oder Tetrahydrofuran eingesetzt werden.

Die Sulfierung kann mit einem molaren Verhältnis von Hydroxycarbonsäureester zu Sulfiermittel von 1 : 0,9 bis 1 : 2,4 durchgeführt werden. Für gesättigte Hydroxycarbonsäureester ist dabei ein Bereich von 1 : 1,0 bis 1 : 1,3, für ungesättigte Hydroxycarbonsäureester ein Bereich von 1 : 1,5 bis 1 : 2,2 bevorzugt.

Die Sulfierung kann bei Temperaturen von 50 bis 98°C durchgeführt werden. Um einerseits eine ausreichend niedrige Viskosität der Einsatzstoffe zu gewährleisten und andererseits eine zu starke thermische Belastung während der Reaktion zu vermeiden, empfiehlt es sich, die Sulfierung zwischen 60 und 90°C durchzuführen.

Die bei der Umsetzung von Hydroxycarbonsäureestern mit Schwefeltrioxid entstehenden sauren Sulfierprodukte werden anschließend in der Art und Weise neutralisiert, daß man Sulfierprodukt und wässrige Basen zusammengibt und dabei einen pH-Wert von 6,5 bis 7,5 einhält. Als Basen für die Neutralisation kommen Alkalimetallhydroxide wie Natrium-, Kalium- und Lithiumhydroxid, Erdalkalimetalloxide und -hydroxide wie Magnesiumoxid, Calciumoxid und Calciumhydroxid, Ammoniak, Mono-, Di- und Tri-$C_{2,4}$-Alkanolamine, beispielsweise Mono-, Di- und Triethanolamin sowie primäre, sekundäre oder tertiäre $C_{1-4}$- Alkylamine in Betracht. Die Neutralisationsbasen kommen dabei bevorzugt in Form mehr oder weniger konzentrierter wässriger Lösungen zum Einsatz, wobei 25 bis 55 gew.-%ige Natriumhydroxidlösungen bevorzugt sind.

Die bei der Sulfierung entstehenden Produkte stellen Sulfate der Hydroxycarbonsäureester dar. Werden Ester ungesättigter Alkohole in die Sulfierung eingesetzt, kann untergeordnet auch eine elektrophile Addition des Schwefeltrioxids an eine oder mehrere Doppelbindungen stattfinden. In diesem Fall resultieren anionische Tenside, die Sulfat- und Sulfonatstruktur aufweisen.

Die erfindungsgemäßen anionischen Tenside können in an sich bekannter Weise durch Zusatz von Wasserstoffperoxid- oder Natriumhypochloritlösung gebleicht werden. Dabei werden, bezogen auf den Feststoffgehalt in der Lösung der Sulfate, 0,2 bis 2,0 Gew.-% Wasserstoffperoxid (berechnet als 100 gew.-%ige Substanz) oder entsprechende Mengen Natriumhypochlorit eingesetzt. Der pH-Wert der Lösungen kann unter Verwendung geeigneter Puffermittel, z. B. mit Natriumphosphat konstant gehalten werden. Zur Stabilisierung gegen Bakterienbefall empfiehlt sich ferner eine Konservierung, z. B. mit Formaldehydlösung, p-Hydroxybenzoat oder anderen bekannten Konservierungsmittel.

Die Produkte zeichnen sich durch hohe Wasserlöslichkeit, gute dermatologische Verträglichkeit und starkes Schaumvermögen aus. Ferner bewirken sie eine deutliche Erniedrigung der Oberflächenspannung des Wassers und fördern die Benetzung fester Grenzflächen mit Wasser.

Die Erfindung betrifft daher ferner die Verwendung der sulfierten Hydroxycarbonsäureester als oberflächenaktive Substanzen, insbesondere zur Herstellung von Wasch- und Reinigungsmitteln.

Die folgenden Beispiele sollen den Gegenstand der Erfindung näher erläutern.

## Beispiele

### Herstellung der Ausgangsverbindungen (Edukte)

Beispiel A:

**Milchsäurelaurylester.** In einem 1-l-Dreihalskolben mit Rührer, Intensivkühler, Wasserabscheider und Innenthermometer wurden 90 g (1 Mol) Milchsäure und 186 g (1 Mol) Laurylalkohol (Lorol[R]) $C_{12}$, Hydroxylzahl 298, Fa.Henkel) vorgelegt und in 200 ml vorgetrocknetem Toluol gelöst. Die Reaktionsmischung wurde mit 5 ml Methansulfonsäure und 14 g (entsprechend 5 % bezogen auf das Gewicht der Ausgangsstoffe) Zinnpulver versetzt und anschließend unter intensivem Rühren und Beaufschlagung mit Inertgas auf 225°C aufgeheizt. Nach dem Ende der Abscheidung von Kondensationswasser, wurde die Veresterung nach 125 min abgebrochen und das Schleppmittel Toluol am Rotationsverdampfer im Wasserstrahlvakuum bei 90°C entfernt. Das rohe Veresterungsprodukt wurde mit Natriumcarbonat neutralisiert und 12 h über Natriumsulfat getrocknet. Nach Abdekantieren wurde das Produkt als klare, hellgelbe Flüssigkeit erhalten und konnte ohne weitere Reinigung in die Sulfierung eingesetzt werden.

Kenndaten des Produktes:

| | |
|---|---|
| Abgeschiedene Menge Wasser | : 16,5 g (92 Gew.-% der Theorie) |
| Hydroxylzahl (OHZ) | : 0,3 |
| Säurezahl (SZ) | : 0,1 |
| Verseifungszahl (VZ) | : 220 |
| Molgewicht (über VZ) | : 258 |

Beispiel B:

**Weinsäuremono-n-octyl-2EO-ester.** Beispiel A wurde mit 150 g (1 Mol) D/L-Weinsäure und 218 g (1 Mol)

eines Anlagerungsproduktes von durchschnittlich 2 Mol Ethylenoxid an n-Octanol (Lorol[R]) $C_8$. Hydroxylzahl 415, Fa. Henkel) wiederholt. Als Katalysatoren für die Veresterung wurden 5 ml Methansulfonsäure und 18,4 g Zinnpulver eingesetzt. Das Produkt fiel als wasserklare Flüssigkeit an.

Kenndaten des Produktes:

| | |
|---|---|
| Abgeschiedene Menge Wasser | : 14,8 g (82 Gew.-% der Theorie) |
| Hydroxylzahl (OHZ) | : 0,5 |
| Säurezahl (SZ) | : 0,1 |
| Verseifungszahl (VZ) | : 160 |
| Molgewicht (über VZ) | : 350 |

**Beispiel C:**

**Citronensäuredioleyl-5EO-ester.** Beispiel A wurde mit 96 g (0,5 Mol) Citronensäure und 488 g (1 Mol) eines Anlagerungsproduktes von durchschnittlich 5 Mol Ethylenoxid an einen technischem Oleylalkohol (HD Ocenol[R]), Iodzahl = 95, Fa.Henkel) in 300 ml vorgetrocknetem Toluol wiederholt. Als Katalysatoren für die Veresterung wurden 8 ml Methansulfonsäure und 29,2 g Zinnpulver eingesetzt. Das Produkt wurde als gelbe, pastöse Flüssigkeit gewonnen.

Kenndaten des Produktes:

| | |
|---|---|
| Abgeschiedene Menge Wasser | : 15,8 g (88 Gew.-% der Theorie) |
| Hydroxylzahl (OHZ) | : 1,3 |
| Säurezahl (SZ) | : 0,1 |
| Iodzahl (IZ) | : 46 |
| Verseifungszahl (VZ) | : 97 |
| Molgewicht (über VZ) | : 1157 |

**Herstellung der erfindungsgemäßen Sulfierprodukte:**

Beispiele 1 - 7:

**Allgemeine Arbeitsvorschrift für die Sulfierung von Hydroxycarbonsäureestern.** In einem 1-l-Sulfierreaktor mit Mantelkühlung und Gaseinleitungsrohr wurden 1 Mol eines Hydroxycarbonsäureesters aus Beispiel A, B oder C vorgelegt und bei einer Temperatur von T = 60 bis 90°C mit 80 bis 160 g (1 bis 2 Mol) gasförmigem Schwefeltrioxid umgesetzt. Das Schwefeltrioxid wurde durch Erhitzen aus einer entsprechenden Menge 65 gew.-%igen Oleums ausgetrieben, mit Stickstoff auf eine Konzentration von 5 Vol.-% verdünnt und innerhalb von 40 bis 80 min in den Hydroxycarbonsäureester eingeleitet. Anschließend wurden die sauren Reaktionsprodukte, die ohne Bleiche als orangegelbe Pasten anfielen, zusammen mit wässriger 25 gew.-%iger Natriumhydroxidlösung neutralisiert, wobei der pH-wert der Lösung bei 6,5 bis 7,5 gehalten wurde. Die Kenndaten der Produkte sind Tab.1 zu entnehmen.

Der Aniontensidgehalt (WAS) und die Unsulfierten Anteile (US) wurden nach den DGF-Einheitsmethoden, Stuttgart, 1950-1984, H-III-10 bzw. G-II-6b ermittelt. Der Sulfatgehalt wurde als Natriumsulfat berechnet, die Bestimmung des Wassergehaltes erfolgte nach der Fischer-Methode.

Tab.1: Sulfatierung von Hydroxycarbonsäureestern
Prozentangaben als Gew.-%

| Bsp. | Edukt | T °C | $SO_3$ Mol | WAS mEq/g | US % | $Na_2SO_4$ % | $H_2O$ % |
|---|---|---|---|---|---|---|---|
| 1 | A | 60 | 1,0 | 0,057 | 6,4 | 1,8 | 71,1 |
| 2 | A | 65 | 1,2 | 0,063 | 5,8 | 1,9 | 71,0 |
| 3 | B | 80 | 1,0 | 0,038 | 6,6 | 2,4 | 73,7 |
| 4 | B | 85 | 1,2 | 0,042 | 6,0 | 2,7 | 73,2 |
| 5 | C | 85 | 1,0 | 0,058 | 8,9 | 2,1 | 80,2 |
| 6 | C | 90 | 1,3 | 0,062 | 8,7 | 2,1 | 69,4 |
| 7 | C | 90 | 2,0 | 0,070 | 6,1 | 2,9 | 70,3 |

Legende: $SO_3$ = Einsatzmenge Mol $SO_3$ pro Mol Ester

## Patentansprüche

1. Anionische Tenside erhältlich aus Hydroxycarbonsäureestern der allgemeinen Formel (I),

$$R^1\text{-O-}(CH_2\overset{\overset{\displaystyle R^5}{|}}{C}HO)_m\text{-CO-}(CH_2)_n\text{-}\underset{\underset{\displaystyle R^2}{|}}{\overset{\overset{\displaystyle HO}{|}}{C}}\text{-}\underset{\underset{\displaystyle R^4}{|}}{\overset{\overset{\displaystyle R^3}{|}}{C}}\text{-H} \qquad (I)$$

in der $R^1$ für einen linearen oder verzweigten aliphatischen Kohlenwasserstoffrest mit 6 bis 22 Kohlenstoffatomen und 0, 1, 2 oder 3 Doppelbindungen, $R^2$ und $R^4$ unabhängig voneinander für Wasserstoff oder eine Gruppe $R^1\text{-O-}(CH_2\text{-}CHR^5\text{-O})_m\text{-CO-}$, $R^3$ für Wasserstoff oder eine Hydroxylgruppe, $R^5$ für Wasserstoff oder eine Methylgruppe, n für 0 oder 1 und m für 0 oder eine Zahl von 1 bis 20 stehen, durch Umsetzung mit einem Sulfiermittel und anschließende Neutralisation mit einer wässrigen Base

2. Anionische Tenside nach Anspruch 1, dadurch gekennzeichnet, daß die Hydroxycarbonsäureester der Formel **(I)** Ester der Citronensäure sind.

3. Anionische Tenside nach Anspruch 1, dadurch gekennzeichnet, daß die Hydroxycarbonsäureester der Formel **(I)** Ester der Weinsäure sind.

4. Anionische Tenside nach mindestens einem der Ansprüche 1 bis 3, dadurch gekennzeichnet, daß sich $R^1$ in Formel **(I)** von linearen, gesättigten Alkoholen mit 8 bis 18 Kohlenstoffatomen ableitet.

5. Anionische Tenside nach mindestens einem der Ansprüche 1 bis 3, dadurch gekennzeichnet, daß sich $R^1$ in Formel **(I)** von ungesättigten Alkoholen mit 16 bis 18 Kohlenstoffatomen und 1, 2 oder 3 Doppelbindungen ableitet.

6. Anionische Tenside nach mindestens einem der Ansprüche 1 bis 4, dadurch gekennzeichnet, daß sich $R^1\text{-O-}(CH_2\text{-}CHR^5\text{-O})_m$ in Formel **(I)** von Anlagerungsprodukten von durchschnittlich 1 bis 20 Mol Ethylenoxid an lineare, gesättigte Alkohole mit 8 bis 18 Kohlenstoffatomen ableitet.

7. Anionische Tenside nach mindestens einem der Ansprüche 1 bis 6, dadurch gekennzeichnet, daß die Sulfierung mit einem molaren Verhältnis von Hydroxycarbonsäureester zu Sulfiermittel von 1 : 0,9 bis 1 : 2,4 durchgeführt wird.

8. Anionische Tenside nach mindestens einem der Ansprüche 1 bis 4 sowie 6 und 7, dadurch gekennzeichnet, daß die Sulfierung mit einem molaren Verhältnis von Hydroxycarbonsäureester zu Sulfiermittel von 1 : 1,1 bis 1 : 1,3 durchgeführt wird.

9. Anionische Tenside nach mindestens einem der Ansprüche 1 bis 3 sowie 5 und 7, dadurch gekennzeichnet, das die Sulfierung mit einem molaren Verhältnis von Hydroxycarbonsäureester zu Sulfiermittel von 1 : 1,5 bis 1 : 2,2 durchgeführt wird.

10. Anionische Tenside nach mindestens einem der Ansprüche 1 bis 9, dadurch gekennzeichnet, daß die Sulfierung bei Temperaturen von 50 bis 98°C durchgeführt wird.

11. Anionische Tenside nach mindestens einem der Ansprüche 1 bis 10, dadurch gekennzeichnet, daß die Sulfierung mit einem $SO_3$/Inertgas-Gemisch durchgeführt wird, das 1 bis 8 Vol.-% Schwefeltrioxid enthält.

12. Anionische Tenside nach mindestens einem der Ansprüche 1 bis 11, dadurch gekennzeichnet, daß die Neutralisation mit wässgen Lösungen von Alkalimetallhydroxiden, Erdalkalimetalloxiden oder -hydroxiden, Ammoniak oder Mono-, Di- oder Tri-$C_{2-4}$-Alkanolaminen durchgeführt wird.

13. Verfahren zur Herstellung anionischer Tenside nach Anspruch 1, dadurch gekennzeichnet, daß Hydroxycarbonsäureestern der allgemeinen Formel **(I)**,

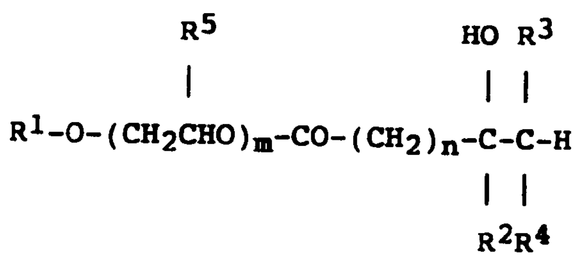

(I)

in der $R^1$ für einen linearen oder verzweigten aliphatischen Kohlenwasserstoffrest mit 6 bis 22 Kohlenstoffatomen und 0, 1, 2 oder 3 Doppelbindungen, $R^2$ und $R^4$ unabhängig voneinander für Wasserstoff oder eine Gruppe $R^1\text{-O-}(CH_2\text{-}CHR^5\text{-O})_m\text{-CO-}$, $R^3$ für Wasserstoff oder eine Hydroxylgruppe, $R^5$ für Wasserstoff oder eine Methylgruppe, n für 0 oder 1 und m für 0 oder eine Zahl von 1 bis 20 stehen, mit einem Sulfiermittel umgesetzt und anschließend mit einer wässrigen Base neutralisiert werden.

14. Verfahren nach Anspruch 13, dadurch gekennzeichnet, daß die Hydroxycarbonsäureester der Formel **(I)** Ester der Citronensäure sind.

15. Verfahren nach Anspruch 13, dadurch gekennzeichnet, daß die Hydroxycarbonsäureester der Formel **(I)** Ester der Weinsäure sind.

16. Verfahren nach mindestens einem der Ansprüche 13 bis 15, dadurch gekennzeichnet, daß sich $R^1$ in Formel **(I)** von linearen, gesättigten Alkoholen mit 8 bis 18 Kohlenstoffatomen ableitet.

**17.** Verfahren nach mindestens einem der Ansprüche 13 bis 15, dadurch gekennzeichnet, daß sich $R^1$ in Formel **(I)** von ungesättigten Alkoholen mit 16 bis 18 Kohlenstoffatomen und 1, 2 oder 3 Doppelbindungen ableitet.

**18.** Verfahren nach mindestens einem der Ansprüche 11 bis 14, dadurch gekennzeichnet, daß sich $R^1$-O-($CH_2$-$CHR^5$-O)$_m$ in Formel **(I)** von Anlagerungsprodukten von durchschnittlich 1 bis 20 Mol Ethylenoxid an lineare, gesättigte aliphatische Alkohole mit 8 bis 18 Kohlenstoffatomen ableitet.

**19.** Verfahren nach mindestens einem der Ansprüche 13 bis 18, dadurch gekennzeichnet, daß die Sulfierung mit einem molaren Verhältnis von Hydroxycarbonsäureester zu Sulfiermittel von 1 : 0,9 bis 1 : 2,4 durchgeführt wird.

**20.** Verfahren nach mindestens einem der Ansprüche 13 bis 16 sowie 16 und 17, dadurch gekennzeichnet, daß die Sulfierung mit einem molaren Verhältnis von Hydroxycarbonsäureester zu Sulfiermittel von 1 : 1,1 bis 1 : 1,3 durchgeführt wird.

**21.** Verfahren nach mindestens einem der Ansprüche 13 bis 15 sowie 15 und 17, dadurch gekennzeichnet, das die Sulfierung mit einem molaren Verhältnis von Hydroxycarbonsäureester zu Sulfiermittel von 1 : 1,5 bis 1 : 2,2 durchgeführt wird.

**22.** Verfahren nach mindestens einem der Ansprüche 13 bis 21, dadurch gekennzeichnet, daß die Sulfierung bei Temperaturen von 50 bis 98°C durchgeführt wird.

**23.** Verfahren nach mindestens einem der Ansprüche 13 bis 22, dadurch gekennzeichnet, daß die Sulfierung mit einem $SO_3$/Inertgas-Gemisch durchgeführt wird, das 1 bis 8 Vol.-% Schwefeltrioxid enthält.

**24.** Verfahren nach mindestens einem der Ansprüche 13 bis 22, dadurch gekennzeichnet, daß die Neutralisation mit wässrigen Lösungen von Alkalimetallhydroxiden, Erdalkalimetalloxiden oder -hydroxiden, Ammoniak oder Mono-, Di- oder Tri-$C_{2-4}$-Alkanolaminen durchgeführt wird.

**25.** Verwendung von anionischen Tensiden nach mindestens einem der Ansprüche 1 bis 11 als oberflächenaktive Substanzen.

**26.** Verwendung nach Anspruch 25, dadurch gekennzeichnet, daß die anionischen Tenside zur Herstellung von Wasch- und Reinigungsmitteln eingesetzt werden.

**27.** Verwendung von anionischen Tensiden hergestellt nach dem Verfahren nach mindestens einem der Ansprüche 13 bis 24 als oberflächenaktive Substanzen.

**28.** Verwendung nach Anspruch 27, dadurch gekennzeichnet, daß die anionischen Tenside zur Herstellung von Wasch- und Reinigungsmitteln eingesetzt werden.

## Claims

**1.** Anionic surfactants obtainable from hydroxycarboxylic acid esters corresponding to general formula (I):

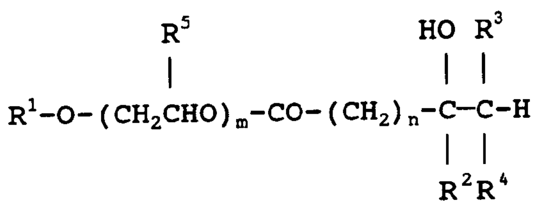

(I)

in which $R^1$ is a linear or branched aliphatic hydrocarbon radical containing 6 to 22 carbon atoms and 0, 1, 2 or 3 double bonds, $R^2$ and $R^4$ independently of one another represent hydrogen or a group $R^1$-O-($CH_2$-$CHR^5$-O)$_m$-CO-, $R^3$ is hydrogen or a hydroxyl group, $R^5$ is hydrogen or a methyl group, n = 0 or 1 and m = 0 or a number of 1 to 20, by reaction with a sulfonating agent and subsequent neutralization with

an aqueous base.

2. Anionic surfactants as claimed in claim 1, characterized in that the hydroxycarboxylic acid esters corresponding to formula (I) are esters of citric acid.

3. Anionic surfactants as claimed in claim 1, characterized in that the hydroxycarboxylic acid esters corresponding to formula (I) are esters of tartaric acid.

4. Anionic surfactants as claimed in at least one of claims 1 to 3, characterized in that $R^1$ in formula (I) is derived from linear saturated alcohols containing 8 to 18 carbon atoms.

5. Anionic surfactants as claimed in at least one of claims 1 to 3, characterized in that $R^1$ in formula (I) is derived from unsaturated alcohols containing 16 to 18 carbon atoms and 1, 2 or 3 double bonds.

6. Anionic surfactants as claimed in at least one of claims 1 to 4, characterized in that $R^1$-O-($CH_2$-$CHR^5$-O)$_m$ in formula (I) is derived from adducts of on average 1 to 20 moles of ethylene oxide with linear saturated alcohols containing 8 to 18 carbon atoms.

7. Anionic surfactants as claimed in at least one of claims 1 to 6, characterized in that the sulfonation reaction is carried out with a molar ratio of hydroxycarboxylic acid ester to sulfonating agent of 1:0.9 to 1:2.4.

8. Anionic surfactants as claimed in at least one of claims 1 to 4 and 6 and 7, characterized in that the sulfonation is carried out with a molar ratio of hydroxycarboxylic acid ester to sulfonating agent of 1:1.1 to 1:1.3.

9. Anionic surfactants as claimed in at least one of claims 1 to 3 and 5 and 7, characterized in that the sulfonation reaction is carried out with a molar ratio of hydroxycarboxylic acid ester to sulfonating agent of 1:1.5 to 1:2.2.

10. Anionic surfactants as claimed in at least one of claims 1 to 9, characterized in that the sulfonation reaction is carried out at temperatures of 50 to 98°C.

11. Anionic surfactants as claimed in at least one of claims 1 to 10, characterized in that the sulfonation reaction is carried out with an $SO_3$/inert gas mixture containing 1 to 8% by volume of sulfur trioxide.

12. Anionic surfactants as claimed in at least one of claims 1 to 11, characterized in that the neutralization is carried out with aqueous solutions of alkali metal hydroxides, alkaline earth metal oxides or hydroxides, ammonia or mono-, di- or tri-$C_{2-4}$-alkanolamines.

13. A process for the production of the anionic surfactants claimed in claim 1, characterized in that hydroxycarboxylic acid esters corresponding to general formula (I):

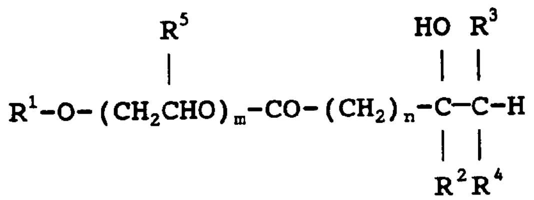

(I)

in which $R^1$ is a linear or branched aliphatic hydrocarbon radical containing 6 to 22 carbon atoms and 0, 1, 2 or 3 double bonds, $R^2$ and $R^4$ independently of one another represent hydrogen or a group $R^1$-O-($CH_2$-$CHR^5$-O)$_m$-CO-, $R^3$ is hydrogen or a hydroxyl group, $R^5$ is hydrogen or a methyl group, n = 0 or 1 and m = 0 or a number of 1 to 20, are reacted with a sulfonating agent and the reaction product is subsequently neutralized with an aqueous base.

14. A process as claimed in claim 13, characterized in that the hydroxycarboxylic acid esters corresponding to formula (I) are esters of citric acid.

15. A process as claimed in claim 13, characterized in that the hydroxycarboxylic acid esters corresponding

to formula (I) are esters of tartaric acid.

16. A process as claimed in at least one of claims 13 to 15, characterized in that $R^1$ in formula (I) is derived from linear saturated alcohols containing 8 to 18 carbon atoms.

17. A process as claimed in at least one of claims 13 to 15, characterized in that $R^1$ in formula (I) is derived from unsaturated alcohols containing 16 to 18 carbon atoms and 1, 2 or 3 double bonds.

18. A process as claimed in at least one of claims 11 to 14, characterized in that $R^1-O-(CH_2-CHR^5-O)_m$ in formula (I) is derived from adducts of on average 1 to 20 moles of ethylene oxide with linear saturated alcohols containing 8 to 18 carbon atoms.

19. A process as claimed in at least one of claims 13 to 18, characterized in that the sulfonation reaction is carried out with a molar ratio of hydroxycarboxylic acid ester to sulfonating agent of 1:0.9 to 1:2.4.

20. A process as claimed in at least one of claims 13 to 16 and 16 and 17, characterized in that the sulfonation is carried out with a molar ratio of hydroxycarboxylic acid ester to sulfonating agent of 1:1.1 to 1:1.3.

21. A process as claimed in at least one of claims 13 to 15 and 15 and 17, characterized in that the sulfonation reaction is carried out with a molar ratio of hydroxycarboxylic acid ester to sulfonating agent of 1:1.5 to 1:2.2.

22. A process as claimed in at least one of claims 13 to 21, characterized in that the sulfonation reaction is carried out at temperatures of 50 to 98°C.

23. A process as claimed in at least one of claims 13 to 22, characterized in that the sulfonation reaction is carried out with an $SO_3$/inert gas mixture containing 1 to 8% by volume of sulfur trioxide.

24. A process as claimed in at least one of claims 13 to 22, characterized in that the neutralization is carried out with aqueous solutions of alkali metal hydroxides, alkaline earth metal oxides or hydroxides, ammonia or mono-, di- or tri-$C_{2-4}$-alkanolamines.

25. The use of the anionic surfactants claimed in at least one of claims 1 to 11 as surface-active substances.

26. The use claimed in claim 25, characterized in that the anionic surfactants are used for the production of detergents and cleaning products.

27. The use of anionic surfactants produced by the process claimed in at least one of claims 13 to 24 as surface-active substances.

28. The use claimed in claim 27, characterized in that the anionic surfactants are used for the production of detergents and cleaning products.

## Revendications

1. Agents tensioactifs anioniques accessibles à partir d'esters d'acide hydrocarboxylique de formule générale (I)

$$R^1-0-(CH_2\overset{R^5}{\overset{|}{C}}HO)_m-CO-(CH_2)_n-\underset{R^2}{\underset{|}{\overset{HO}{\overset{|}{C}}}}-\underset{R^4}{\underset{|}{\overset{R^3}{\overset{|}{C}}}}-H \qquad (I)$$

dans laquelle $R_1$ représente un radical hydrocarboné aliphatique, linéaire ou ramifié, ayant de 6 à 22 atomes de carbone et 0,1, 2 ou 3 doubles liaisons,
$R^2$ et $R^4$ , indépendamment l'un de l'autre, représentent de l'hydrogène ou un groupe $R^1-0-(CH_2-CHR^5-$

$O)_m$-CO-
$R^3$ représente de l'hydrogène ou un groupe hydroxyle,
$R^5$ représente de l'hydrogène ou un groupe méthyle,
n représente 0 ou 1,
et m représente 0 ou un nombre allant de 1 à 20,
par réaction avec un agent de sulfonation et neutralisation corrélative avec une base aqueuse.

**2.** Agents tensioactifs anioniques selon la revendication 1, caractérisés en ce que les esters d'acide hydroxycarboxylique de formule (I) sont des esters d'acide citrique.

**3.** Agents tensioactifs anioniques selon la revendication 1, caractérisés en ce que les esters d'acide hydroxycarboxylique de formule (I) sont des esters d'acide tartrique.

**4.** Agents tensioactifs anioniques selon au moins une des revendications 1 à 3, caractérisés en ce que $R^1$ dans la formule (I) dérive d'alcools saturés, linéaires, ayant de 8 à 18 atomes de carbone.

**5.** Agents tensioactifs anioniques selon au moins une des revendications 1 à 3, caractérisés en ce que $R^1$ dans la formule (I) dérive d'alcools non saturés ayant de 16 à 18 atomes de carbone et une, deux ou trois doubles liaisons.

**6.** Agents tensioactifs anioniques selon au moins une des revendications 1 à 4, caractérisés en ce que $R^1$-O-$CH_2$-$CHR^5$-$O)_m$ dans la formule (I) dérive de produits d'addition de 1 à 20 mol en moyenne d'oxyde d'éthylène sur des alcools linéaires, saturés, ayant de 8 à 18 atomes de carbone.

**7.** Agents tensioactifs anioniques selon au moins une des revendications 1 à 6, caractérisés en ce que la sulfonation est effectuée avec un rapport molaire de l'ester d'acide hydroxycarboxylique à l'agent de sulfonation allant de 1 : 0,9 à 1 : 2,4.

**8.** Agents tensioactifs anioniques selon au moins une des revendications 1 à 4 ainsi que 6 et 7, caractérisés en ce que la sulfonation est effectuée avec un rapport molaire de l'ester d'acide hydroxycarboxylique à l'agent de sulfonation allant de 1 : 1,1 à 1 : 1,3.

**9.** Agents tensioactifs anioniques selon au moins une des revendications 1 à 3 ainsi que 5 et 7, caractérisés en ce que la sulfonation est effectuée avec un rapport molaire de l'ester d'acide hydroxycarboxylique à l'agent de sulfonation allant de 1 : 1,5 à 1 : 2,2.

**10.** Agents tensioactifs anioniques selon au moins une des revendications 1 à 9, caractérisés en ce que la sulfonation est effectuée à des températures allant de 50 à 98°C.

**11.** Agents tensioactifs anioniques selon au moins une des revendications 1 à 10, caractérisés en ce que la sulfonation est effectuée avec un mélange $SO_3$/gaz inerte qui renferme de 1 à 8 % en volume d'anhydride sulfurique.

**12.** Agents tensioactifs anioniques selon au moins une des revendications 1 à 11, caractérisés en ce que la neutralisation est effectuée avec des solutions aqueuses d'hydroxyde de métal alcalin, d'oxydes ou d'hydroxydes de métal alcalino-terreux, d'ammoniac ou de mono-, di- ou trialcanolamines en $C_2$ à $C_4$.

**13.** Procédé d'obtention d'agents tensioactifs anioniques selon la revendication 1, caractérisé en ce que des esters d'acide hydroxycarboxylique de formule générale (I)

$$\begin{array}{ccccc} & R^5 & & HO & R^3 \\ & | & & | & | \\ R^1\text{-O-}(CH_2 & CHO)_m\text{-CO-}(CH_2)_n & - & C & -C-H \\ & & & | & | \\ & & & R^2 & R^4 \end{array} \qquad (I)$$

dans laquelle $R_1$ représente un radical hydrocarboné aliphatique, linéaire ou ramifié, ayant de 6 à 22 atomes de carbone et 0,1, 2 ou 3 doubles liaisons,

$R^2$ et $R^4$ , indépendamment l'un de l'autre, représentent de l'hydrogène ou un groupe $R^1$-0-($CH_2$-$CHR^5$-0)$_m$-CO-,
$R^3$ représente de l'hydrogène ou un radical hydroxyle,
$R^5$ représente de l'hydrogène ou un radical méthyle,
n représente 0 ou 1 et m représente 0 ou un nombre allant de 1 à 20,
sont mis à réagir avec un agent de sulfonation et ensuite sont neutralisés avec une base aqueuse.

**14.** Procédé selon la revendication 13, caractérisé en ce que les esters d'acide hydroxycarboxylique de formule (I) sont des esters de l'acide citrique.

**15.** Procédé selon la revendication 13, caractérisé en ce que les esters d'acide hydroxycarboxylique de formule (I) sont des esters d'acide tartrique.

**16.** Procédé selon au moins une des revendications 13 à 15, caractérisé en ce que $R^1$ dans la formule (I) dérive des alcools saturés, linéaires ayant de 8 à 18 atomes de carbone.

**17.** Procédé selon au moins une des revendications 13 à 15, caractérisé en ce que $R^1$ dans la formule (I) dérive d'alcools non saturés ayant de 16 à 18 atomes de carbone et 1, 2 ou 3 doubles liaisons.

**18.** Procédé selon au moins une des revendications 11 à 14, caractérisé en ce que $R^1$-0-($CH_2$-$CHR^5$-0)$_m$ dans la formule (I) dérive de produits d'addition de 1 à 20 mol d'oxyde d'éthylène en moyenne sur des alcools aliphatiques saturés, linéaires ayant de 8 à 18 atomes de carbone.

**19.** Procédé selon au moins une des revendications 13 à 18, caractérisé en ce que la sulfonation est effectuée avec un rapport molaire de l'ester d'acide hydroxycarboxylique à l'agent de sulfonation de 1 : 0,9 à 1 : 2,4.

**20.** Procédé selon au moins une des revendications 13 à 16, ainsi que 16 et 17, caractérisé en ce que la sulfonation est effectuée avec un rapport molaire de l'ester d'acide hydroxycarboxylique à l'agent de sulfonation allant de 1 : 1,1 à 1 : 1,3.

**21.** Procédé selon au moins une des revendications 13 à 15, ainsi que 15 et 17, caractérisé en ce que la sulfonation est effectuée avec un rapport molaire de l'ester d'acide hydroxycarboxylique à l'agent de sulfonation, allant de 1 : 1,5 à 1 : 2,2.

**22.** Procédé selon au moins une des revendications 13 à 21, caractérisé en ce que la sulfonation est effectuée à des températures de 50 à 98°C.

**23.** Procédé selon au moins une des revendications 13 à 22, caractérisé en ce que la sulfonation est effectuée avec un mélange $SO_3$/gaz inerte qui renferme de 1 à 8 % en volume d'anhydride sulfurique.

**24.** Procédé selon au moins une des revendications 13 à 22, caractérisé en ce que la neutralisation est effectuée avec des solutions aqueuses d'hydroxydes de métal alcalin, d'oxydes ou d'hydroxydes de métal alcalino-terreux, d'ammoniac, ou de mono-, di- ou trialcanolamines en $C_2$-$C_4$.

**25.** Utilisation d'agents tensioactifs anioniques selon au moins une des revendications 1 à 11, comme substances tensioactives;

**26.** Utilisation selon la revendication 25, caractérisée en ce que les agents tensioactifs anioniques sont mis en oeuvre en vue de la production d'agents de lavage et de nettoyage.

**27.** Utilisation d'agents tensioactifs non ioniques obtenus selon le procédé, selon au moins une des revendications 13 à 24, comme substances tensioactives.

**28.** Utilisation selon la revendication 27, caractérisée en ce que les agents tensioactifs anioniques sont mis en oeuvre en vue de la production d'agents de lavage et de nettoyage.